# FASCICULE DE BREVET EUROPEEN

(11) **EP 2 066 768 B1**
(45) Date de publication et mention de la délivrance du brevet: **18.05.2016**
(21) Numéro de dépôt: 07858385.3
(22) Date de dépôt: 27.09.2007
(51) Int. Cl.: C07C 67/08, C07C 69/732, C07C 69/675, C11C 3/00, C10M 105/32, C10M 105/36, C10M 105/40

(54) **PROCÉDÉ DE SYNTHÈSE D'ESTERS D'ESTOLIDES**
VERFAHREN ZUR SYNTHESE VON ESTOLIDESTERN
METHOD FOR THE SYNTHESIS OF ESTOLIDE ESTERS

(30) Priorité: 29.09.2006 FR 0608600
(43) Date de publication de la demande: 10.06.2009
(73) Titulaire: Stearinerie Dubois Fils, Societe Anonyme, 36300 Ciron (FR)
(72) Inventeur: BRISSAUD, Sandrine, F-36200 Argenton sur Creuse (FR); BAUDRY, Mathieu, F-92100 Boulogne-Billancourt (FR); PIERRE, Ronan, F-59830 Cysoing (FR)
(74) Mandataire: Hammond, William
(86) Numéro de dépôt international: PCT/FR2007/001583
(87) Numéro de publication internationale: WO 2008/040864

(56) Documents cités:
- EP-A1- 0 000 424
- WO-A-2004/041769
- JP-A- 7 228 881
- US-A- 4 428 885
- CERMAK ET AL: "Synthesis and physical properties of estolides from lesquerella and castor fatty acid esters" INDUSTRIAL CROPS AND PRODUCTS, ELSEVIER, NL, vol. 23, no. 1, janvier 2006 (2006-01), pages 54-64, XP005259161 ISSN: 0926-6690

## Description

La présente invention propose un procédé de synthèse monotope, plus connu sous sa désignation anglaise « *one-pot »,* et sans solvant toxique d'esters d'estolides saturés de degré d'oligomérisation élevé et contrôlé et de faible indice d'acide résiduel.

Les corps gras d'origine végétale sont connus depuis fort longtemps pour leurs propriétés lubrifiantes. Cependant, leur utilisation industrielle est limitée par leur faible stabilité thermo-oxydative.

Le marché des lubrifiants est donc naturellement dominé par des huiles minérales et, dans une moindre mesure, par des esters synthétiques.

Cependant, on assiste actuellement à un intérêt accru pour les produits biodégradables ou d'origine végétale, suite notamment à l'apparition d'écolabels (Blue Angel en Allemagne par exemple) et aux récents durcissements de la réglementation relative aux lubrifiants.

Par ailleurs, les hausses du cours du pétrole et les tensions internationales non sans conséquences sur l'approvisionnement en pétrole conduisent notamment les industriels à étudier des produits de substitution à ceux issus du pétrole.

C'est pourquoi, une attention toute particulière est portée aux estolides en particulier ceux d'origine végétale. En effet, généralement estérifiés, les estolides sont des polymères carbonés biodégradables. De plus lorsqu'ils sont saturés, les esters d'estolides présentent une stabilité thermo-oxydative élevée supérieure à celle d'autres esters d'origine végétale. L'ajout d'additifs adaptés leur permet d'atteindre des performances comparables à celles des huiles minérales additivées. Ces caractéristiques en font des cibles de choix pour le secteur des lubrifiants automobiles et industriels où leurs propriétés lubrifiantes et anti-usure sont également appréciées.

Les estolides sont aussi utilisés en cosmétique pour leurs propriétés émollientes et hydratantes et leurs esters de polyols y sont utilisés comme tensioactifs.

De nombreux procédés d'obtention d'esters d'estolides ou d'estolidation sont connus de l'homme de l'art. En fait, on peut les regrouper en trois groupes.

Le premier consiste à oligomériser des acides carboxyliques hydroxylés. La réaction est une réaction d'estérification entre la fonction acide d'un hydroxyacide et la fonction hydroxyle d'une autre molécule d'hydroxyacide : 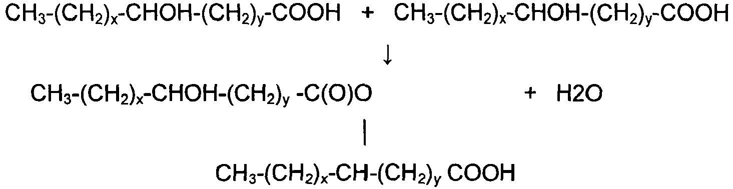 Cette réaction peut être poursuivie jusqu'au degré d'oligomérisation souhaité.

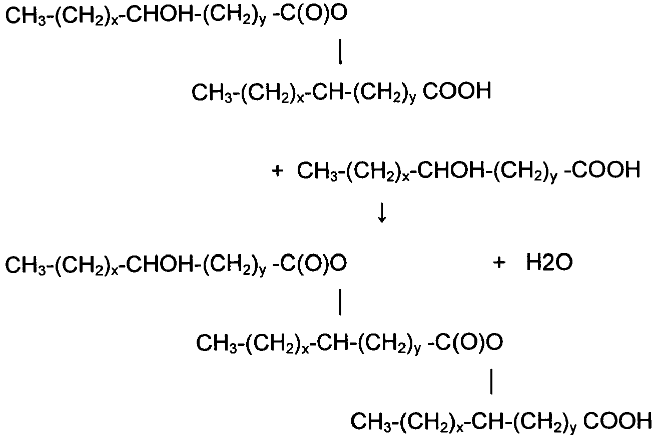

Dans ces formules, x et y sont chacun compris entre 0 et 20.

En variante, il peut aussi s'agir de la réaction d'estérification entre la fonction acide carboxylique d'un acide non hydroxylé et la fonction hydroxyle d'un hydroxyacide. Cependant, cette réaction ne permet pas d'obtenir d'oligomères:

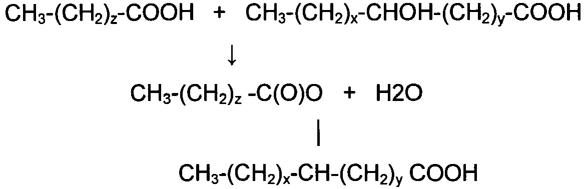

Dans ces formules, x, y et z sont chacun compris entre 0 et 20.

Cette réaction est généralement réalisée à une température comprise entre 100 et 240°C, sous atmosphère inerte et en présence de catalyseur. La réaction étant équilibrée, elle est favorisée en éliminant l'eau formée lors de la réaction par différents procédés connus de l'homme de l'art. Il est par exemple possible de travailler sous pression réduite mais la méthode couramment utilisée dans le cas des estolides est l'utilisation d'un solvant formant un azéotrope avec l'eau comme le xylène.

Les acides carboxyliques hydroxylés les plus utilisés sont les acides gras d'origine végétale comme l'acide ricinoléique, hydroxystéarique ou lesquerolique mais les matières premières qui peuvent être utilisées ne se limitent pas à ces exemples. On peut aussi envisager l'utilisation d'acides plus courts comme l'acide lactique ou d'acides polyhydroxylés et de polyacides hydroxylés.

Le deuxième groupe de procédés pour synthétiser des estolides consiste en la polymérisation, en milieu acide, d'acides insaturés qui conduit elle aussi au même type de molécules. Il ne s'agit cependant pas d'une estérification à proprement parler mais d'une polymérisation mettant en jeu les insaturations des acides gras. Les acides utilisés sont alors préférentiellement choisis parmi les acides gras insaturés d'origine végétale comme l'acide oléique.

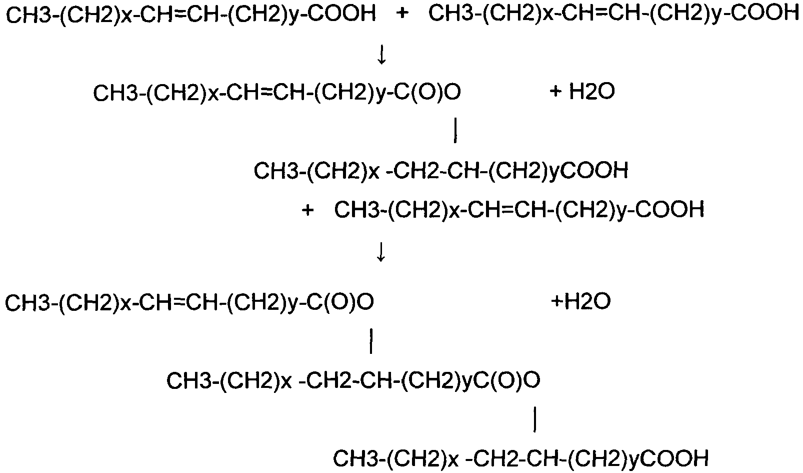

Dans ces formules, x et y sont chacun compris entre 0 et 20

Enfin, le troisième groupe comprend des procédés de synthèse qui conduisent directement à un ester d'estolide. Ils consistent à époxyder un ester d'acide gras insaturé comme un ester méthylique d'acide gras de tournesol oléique, puis à le faire réagir avec un autre acide gras (X. Pages et coll., Oléagineux Corps gras Lipides, Vol.8 N°2, mars/avril 2001). Cependant cela ne permet pas non plus d'obtenir des oligomères et l'ester obtenu présente une fonction hydroxyle supplémentaire.

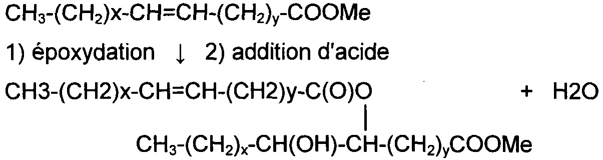

Dans ces formules, x et y sont chacun compris entre 0 et 20.

Les documents WO-9215548-A, WO-9925794-A, et WO-0153247-A décrivent la synthèse d'esters d'estolides destinés à l'industrie des lubrifiants. La réaction est réalisée en plusieurs étapes. Dans un premier temps, elle consiste à polymériser les insaturations d'un acide gras insaturé, en particulier l'acide oléique, en milieu acide sulfurique avant d'estérifier l'estolide formé avec un alcool ramifié. Cette méthode présente l'inconvénient de conduire à un dérivé partiellement insaturé, ce qui n'est pas souhaitable pour les applications dans le domaine des lubrifiants car les insaturations réduisent la stabilité thermo-oxydative. Ce problème est résolu en ajoutant une étape intermédiaire qui consiste à faire réagir un acide gras saturé sur l'insaturation de la chaîne acide terminale de l'estolide. La méthode conduit donc à des esters d'estolides aux propriétés intéressantes mais après 3 étapes et les dérivés obtenus sont très colorés (10 à 17 Gardner).

Le document JP 7228881 concerne aussi des esters d'estolides utilisables comme lubrifiants mais il s'agit d'une réaction d'estolidation en présence de xylène, solvant toxique, suivie de l'estérification de l'estolide formé.

Plusieurs documents décrivent la synthèse d'esters d'estolides et de polyols pouvant servir de tensioactifs. Ainsi, les documents EP-0000424-A et US-2006/0041158-A concernent en particulier la synthèse de polyhydroxystérate de polyéthylèneglycol. Dans les deux cas, le procédé consiste à faire réagir simultanément l'acide hydroxystéarique et le polyol afin de former l'ester d'estolide. Cependant, les indices d'acide des esters obtenus sont élevés (supérieur à 5 pour le premier) ce qui n'est pas souhaitable pour les lubrifiants et, de plus, le procédé décrit dans le document EP-0000424-A a recours au xylène.

Le document US-4428885-A décrit la synthèse d'esters d'estolides et de stérol. La synthèse des esters est réalisée en 2 étapes. L'acide hydroxystéarique ou ricinoléique est tout d'abord oligomérisé puis l'estolide obtenu est estérifié avec le cholestérol. Les deux étapes sont réalisées en présence de xylène. Dans ce document est aussi évoquée la possibilité de réaliser la synthèse en une seule étape mais alors un faible excès d'acide hydroxystéarique (50%) est utilisé, ce qui ne conduit qu'à environ 50% d'estolide.

Aussi, un des buts de la présente invention est-il de fournir un procédé pour synthétiser des esters d'estolides de degré d'oligomérisation élevé et contrôlé et de faible indice d'acide.

Un autre but est de fournir un tel procédé qui ne présente pas les inconvénients décrits précédemment.

Ces buts, ainsi que d'autres qui apparaîtront par la suite, sont atteints par un procédé pour synthétiser des esters d'estolides de degré d'oligomérisation élevé et contrôlé et de faible indice d'acide résiduel, qui est, selon la présente invention, caractérisé par le fait qu'il comporte simultanément une oligomérisation d'un hydroxacide saturé et une estérification de cet hydroxyacide par un monoalcool en absence de solvant.

Avantageusement, l'hydroxyacide est choisi parmi les acides carboxyliques saturés et hydroxylés, tels que des acides carboxyliques saturés et monohydroxylés.

De préférence, les acides carboxyliques saturés et hydroxylés sont des acides carboxyliques saturés et monohydroxylés d'origine végétale tels que l'acide 12-hydroxystéarique.

Selon un autre mode de réalisation, l'hydroxyacide est choisi parmi les acides hydroxylés, les acides polyhydroxylés et les polyacides hydroxylés.

Avantageusement, l'alcool est choisi parmi les monoalcools et en particulier parmi les monoalcools ramifiés comme par exemple l'éthyl-2-hexanol.

De préférence, le procédé selon la présente invention est réalisé à une température comprise entre 100 et 200°C, et plus précisément entre 140 et 180°C.

Avantageusement, ce procédé est réalisé en présence d'un gaz inerte tel que l'azote ou l'argon.

De préférence, on ajoute un catalyseur choisi parmi les acides minéraux ou organiques, les bases minérales ou organiques les catalyseurs à base d'étain ou de titane.

Avantageusement, le catalyseur est l'acide paratoluènesulfonique ou l'acide méthanesulfonique.

De préférence, on conduit ce procédé jusqu'à obtenir un indice d'acide inférieur ou égal à 5, et préférentiellement inférieur à 3.

La description qui va suivre, et qui ne présente aucun caractère limitatif, permettra à l'homme du métier de mieux comprendre la mise en oeuvre de la présente invention ainsi que les avantages de celle-ci.

Les hydroxyacides utilisés sont des acides carboxyliques saturés et hydroxylés, préférentiellement des acides carboxyliques saturés et monohydroxylés et plus préférentiellement des acides carboxyliques saturés et monohydroxylés d'origine végétale tels que l'acide 12-hydroxystéarique.

Il est cependant possible d'utiliser d'autres types d'acides hydroxylés, tels que des acides hydroxylés d'origine synthétique, des acides polyhydroxylés ou des polyacides hydroxylés.

Les alcools utilisés sont des monoalcools, de préférence des monoalcools ramifiés tels que l'éthyl-2-hexanol, ou des alcools isoalkyliques.

La réaction consiste à faire réagir sous agitation dans un réacteur correctement dimensionné une quantité définie d'hydroxyacide et d'alcool en fonction du degré d'oligomérisation souhaité.

Il est préférable d'introduire la totalité de l'hydroxyacide et de l'alcool dans le réacteur dès le début de la réaction.

Selon une variante, on introduit initialement une quantité équimolaire d'hydroxyacide et d'alcool afin de former dans un premier temps un hydroxyester. Le reste de l'hydroxyacide est ensuite ajouté dans le réacteur afin de former l'ester d'estolide recherché. Cette seconde méthode, bien que fractionnée conduit aux mêmes dérivés et reste conforme à l'esprit de l'invention puisque aucun traitement intermédiaire n'est réalisé sur le produit.

La réaction est réalisée à une température comprise entre 100 et 200°C, préférentiellement à une température comprise entre 140 et 180°C.

La réaction peut être réalisée sous pression réduite ou à pression atmosphérique et éventuellement en présence d'un gaz inerte tel que l'azote ou l'argon. La réaction sera de préférence réalisée à pression atmosphérique et sous flux de gaz inerte.

La réaction peut être accélérée par l'ajout d'un catalyseur. Les catalyseurs qui peuvent être utilisés sont hétérogènes ou homogènes tels que les acides minéraux ou organiques usuels (acide sulfurique, acide phosphorique, acide paratoluènesulfonique, ...), les bases minérales ou organiques (soude, potasse, alcoolates alcalins et alcalino-terreux,...), les catalyseurs à base d'étain (oxyde d'étain,...) ou de titane (tétrabutyltitanate, tétraisopropyltitanate,...) ; mais le choix du catalyseur n'est pas limité aux catalyseurs ci-dessus. La réaction sera cependant préférentiellement réalisée en présence d'un catalyseur acide tel que l'acide paratoluènesulfonique ou l'acide méthanesulfonique.

La réaction est poursuivie jusqu'à oligomérisation totale de l'acide hydroxylé ou jusqu'à obtention de l'indice d'acide souhaité. On poursuivra de préférence la réaction jusqu'à obtenir un indice d'acide inférieur ou égal à 5, et préférentiellement inférieur à 3 en fonction des applications souhaitées.

En fin de réaction, l'ester d'estolide obtenu peut-être filtré sans traitement supplémentaire. Cependant, on réalisera préférentiellement une neutralisation du catalyseur si un catalyseur homogène a été utilisé. Cette neutralisation pourra être suivie d'une étape de séchage sous vide de l'ester pour éliminer l'eau résiduelle.

L'objet de l'invention est illustré par les exemples suivants.

### Exemple 1 :

195 g d'éthyl-2-hexanol (1,5 mol), 450 g d'acide 12-hydroxystéarique (1,5 mol) et 1,5 g d'acide méthanesulfonique sont introduits dans un réacteur en verre de 2 litres. Le milieu réactionnel est chauffé à 145°C sous azote et sous agitation mécanique pendant 2 heures. 900 g d'acide 12-hydroxystéarique (3,0 mol) et 1,5 g d'acide méthanesulfonique sont ensuite ajoutés et le milieu réactionnel est chauffé sous azote à 150°C pendant 7h30 jusqu'à obtenir un indice d'acide inférieur à 2. Le catalyseur est ensuite neutralisé avec 0,8 g de soude et le milieu réactionnel est lavé à l'eau puis séché à 90°C sous vide et filtré.

### Exemple 2 :

195 g d'éthyl-2-hexanol (1,5 mol), 1350 g d'acide 12-hydroxystéarique (4,5 mol) et 3 g d'acide méthanesulfonique sont introduits dans un réacteur en verre de 2 litres. Le milieu réactionnel est chauffé à 145°C sous azote et sous agitation mécanique pendant 10 heures jusqu'à obtenir un indice d'acide inférieur à 2. Le catalyseur est ensuite neutralisé avec 1,2 g de soude et le milieu réactionnel est lavé à l'eau puis séché à 90°C sous vide et filtré.

### Exemple 3 :

300 g d'isotridécanol (1,5 mol), 1350 g d'acide 12-hydroxystéarique (4,5 mol) et 3,3 g d'acide méthanesulfonique sont introduits dans un réacteur en verre de 2 litres. Le milieu réactionnel est chauffé à 145°C sous azote et sous agitation mécanique pendant 7 heures jusqu'à obtenir un indice d'acide inférieur à 2. Le catalyseur est ensuite neutralisé avec 1,0 g de soude et le milieu réactionnel est lavé à l'eau puis séché à 90°C sous vide et filtré.

### Exemple 4 :

195 g d'éthyl-2-hexanol (1,5 mol), 1800 g d'acide 12-hydroxystéarique (6 mol) et 4 g d'acide méthanesulfonique sont introduits dans un réacteur en verre de 2 litres. Le milieu réactionnel est chauffé à 145°C sous azote et sous agitation mécanique pendant 9 heures jusqu'à obtenir un indice d'acide inférieur à 3. Le catalyseur est ensuite neutralisé avec 1,5 g de soude et le milieu réactionnel est lavé à l'eau puis séché à 90°C sous vide et filtré.

### Exemple 5 :

130 g d'éthyl-2-hexanol (1,0 mol), 1500 g d'acide 12-hydroxystéarique (5 mol) et 3,3 g d'acide méthanesulfonique sont introduits dans un réacteur en verre de 2 litres. Le milieu réactionnel est chauffé à 150°C sous azote et sous agitation mécanique pendant 13 heures jusqu'à obtenir un indice d'acide inférieur à 3. Le catalyseur est ensuite neutralisé avec 1,5 g de soude et le milieu réactionnel est lavé à l'eau puis séché à 90°C sous vide et filtré.

### Exemple 6 :

237 g d'isodécanol (1,5 mol), 1350 g d'acide 12-hydroxystéarique (4,5 mol) et 3,2 g d'acide méthanesulfonique sont introduits dans un réacteur en verre de 2 litres. Le milieu réactionnel est chauffé à 150°C sous azote et sous agitation mécanique pendant 7 heures 30 jusqu'à obtenir un indice d'acide inférieur à 3. Le catalyseur est ensuite neutralisé avec 0,5 g de soude et le milieu réactionnel est lavé à l'eau puis séché à 90°C sous vide et filtré.

### Exemple 7 :

363 g d'alcool isocétylique (1,5 mol), 1350 g d'acide 12-hydroxystéarique (4,5 mol) et 3,4 g d'acide méthanesulfonique sont introduits dans un réacteur en verre de 2 litres. Le milieu réactionnel est chauffé à 150°C sous azote et sous agitation mécanique pendant 5 heures jusqu'à obtenir un indice d'acide inférieur à 3. Le catalyseur est ensuite neutralisé avec 1,75 g de soude et le milieu réactionnel est lavé à l'eau puis séché à 90°C sous vide et filtré.

Les analyses des produits obtenus dans ces exemples 1 à 7 sont regroupées dans le Tableau ci-après.

Les esters ainsi obtenus sont de couleur jaune pâle à ambre, de faible indice d'acide (IA≤2), hautement saturés (II < 3) et de point de trouble et de point d'écoulement inférieurs à 0°C ce qui convient particulièrement pour des applications dans le domaine des lubrifiants. Les viscosités de ces esters illustrent bien le fait que la viscosité peut être ajustée grâce à la maîtrise du degré d'oligomérisation de l'estolide (cf. Tableau)

Le procédé pour synthétiser des esters d'estolides présente l'avantage de combiner dans un seul réacteur l'estolidation et l'estérification sans aucun traitement intermédiaire. Ce procédé n'implique aucun traitement de l'estolide (décoloration, filtration,) avant l'estérification, ce qui permet de supprimer une source de déchets et de gagner du temps. Cette synthèse est réalisée sans solvant ce qui permet d'éviter une étape d'élimination du solvant et de supprimer les coûts de destruction ou de retraitement de celui-ci. Il s'agit aussi d'un gain au niveau de la sécurité, les solvants utilisés étant généralement des solvants toxiques comme le xylène.

Ce procédé selon l'invention permet également de maîtriser le degré d'oligomérisation de l'hydroxyacide et par conséquent la viscosité de l'ester en contrôlant dès le départ la stoechiométrie alcool/ hydroxyacide.

Enfin, la voie de synthèse proposée conduit à des esters d'estolides saturés et de faible indice d'acide, propriétés recherchées notamment dans le domaine des lubrifiants.

**Tableau**

| *exemple* | *Alcool utilisé* | *Degré d'oligo.* | *Ajout fractionné de l'acide* | *Viscosité cinématique 40°C* | *Isapo* | *IOH* | *II* | *IA* | *Pt trouble*/ *écoulement* | *Couleur (Gardner)* | *V.I.* |
|---|---|---|---|---|---|---|---|---|---|---|---|
| N°1 | E2H | 3 | OUI | 265 cSt | 175 | 25 | 3,0 | 1,1 | <0°C | 5,4 G | > 160 |
| N°2 | E2H | 3 | NON | 245 cSt | 175 | 33 | 2,3 | 0,3 | <0°C | 7,6 G | > 160 |
| N°3 | oxo13 | 3 | NON | 255 cSt | 163 | 35 | 1,8 | 0,35 | <0°C | 5,5 G | > 160 |
| N°4 | E2H | 4 | NON | 455 cSt | 182 | 26 | 2,2 | 1,1 | <0°C | 5,7 G | > 160 |
| N°5 | E2H | 5 | NON | 800 cSt | 186 | 23 | 2,2 | 1,5 | <0°C | 6,5 G | > 160 |
| N°6 | oxo10 | 3 | NON | 235 cSt | 172 | 30 | 1,8 | 0,3 | <0°C | 5,1 G | - |
| N°7 | Isocet. | 3 | NON | 265 cSt | 156 | 34 | 1,4 | 0,7 | <0°C | 7,0 G | - |

| | | | | | | | | | | | |
|---|---|---|---|---|---|---|---|---|---|---|---|
| Abréviations : E2H : éthyl-2-hexanol oxo13 : isotridécanol oxo10 : isodécanol Isocet. : alcool isocétylique Degré d'oligo. : degré d'oligomérisation de l'acide hydroxylé Isapo : Indice de saponification IOH : Indice d'hydroxyle II : Indice d'iode IA : indice d'acide Pt trouble/écoulement : point de trouble et point d'écoulement V.I. : Indice de viscosité cinématique | | | | | | | | | | | |

## Revendications

1. Procédé monotope, ou « one-pot », pour synthétiser des esters d'estolides, **caractérisé par le fait qu'**on estérifie en absence de solvant un hydroxyacide saturé par un monoalcool et, que l'on réalise simultanément une oligomérisation pour former des esters d'estolides présentant un degré d'oligomérisation supérieur ou égal à 3, un indice d'acide inférieur ou égal à 5 et qui sont saturés et thermo-oxydativement stables.

2. Procédé selon la revendication 1, **caractérisé par le fait que** l'hydroxyacide est choisi parmi les acides carboxyliques saturés et hydroxylés.

3. Procédé selon la revendication 2, **caractérisé par le fait que** les acides carboxyliques saturés et hydroxylés sont des acides carboxyliques d'origine végétale tels que l'acide 12-hydroxystéarique.

4. Procédé selon la revendication 1, **caractérisé par le fait que** le monoalcool est choisi parmi les monoalcools ramifiés.

5. Procédé selon la revendication 4, **caractérisé par le fait que** l'alcool est l'éthyl-2-hexanol.

6. Procédé selon la revendication 4, **caractérisé par le fait que** l'alcool est choisi parmi les alcools isoalkyliques.

7. Procédé selon l'une quelconque des revendications 1 à 6, **caractérisé par le fait qu'**il est réalisé à une température comprise entre 100 et 200°C.

8. Procédé selon la revendication 7, **caractérisé par le fait qu'**il est réalisé à une température comprise entre 140 et 180°C.

9. Procédé selon l'une quelconque des revendications 1 à 8, **caractérisé par le fait qu'**il est réalisé en présence d'un gaz inerte tel que l'azote ou l'argon.

10. Procédé selon l'une quelconque des revendications 1 à 9, **caractérisé par le fait qu'**on ajoute un catalyseur.

11. Procédé selon la revendication 10, **caractérisé par le fait que** le catalyseur est choisi parmi les acides minéraux ou organiques, les bases minérales ou organiques les catalyseurs à base d'étain ou de titane.

12. Procédé selon la revendication 10, **caractérisé par le fait que** le catalyseur est l'acide paratoluènesulfonique ou l'acide méthanesulfonique.

13. Procédé selon l'une quelconque des revendications 1 à 12, **caractérisé par le fait que** l'on obtient un indice d'acide inférieur ou égal à 3.

## Patentansprüche

1. Monotopes Verfahren oder "Eintopfverfahren" zur Synthese von Estolidestern, **dadurch gekennzeichnet, dass** eine gesättigte Hydroxysäure in Abwesenheit eines Lösungsmittels durch einen Monoalkohol verestert wird und dass gleichzeitig eine Oligomerisierung durchgeführt wird, um Estolidester zu bilden, die einen Oligomerisierungsgrad größer oder gleich 3 aufweisen, eine Säurezahl kleiner oder gleich 5 aufweisen und gesättigt sind und thermo-oxidativ stabil sind.

2. Verfahren nach Anspruch 1, **dadurch gekennzeichnet, dass** die Hydroxysäure ausgewählt wird aus den gesättigten und hydroxilierten Carbonsäuren.

3. Verfahren nach Anspruch 2, **dadurch gekennzeichnet, dass** die gesättigten und hydroxilierten Carbonsäuren solche pflanzlichen Ursprungs sind, wie 12-Hydroxystearinsäure.

4. Verfahren nach Anspruch 1, **dadurch gekennzeichnet, dass** der Monoalkohol aus den verzweigten Monoalkoholen ausgewählt wird.

5. Verfahren nach Anspruch 4, **dadurch gekennzeichnet, dass** der Alkohol 2-Ethylhexanol ist.

6. Verfahren nach Anspruch 4, **dadurch gekennzeichnet, dass** der Alkohol ausgewählt wird aus den Isoalkylalkoholen.

7. Verfahren nach einem der Ansprüche 1 bis 6, **dadurch gekennzeichnet, dass** dieses bei einer Temperatur zwischen 100 und 200°C durchgeführt wird.

8. Verfahren nach Anspruch 7, **dadurch gekennzeichnet, dass** dieses bei einer Temperatur zwischen 140 und 180°C durchgeführt wird.

9. Verfahren nach einem der Ansprüche 1 bis 8, **dadurch gekennzeichnet, dass** dieses in Anwesenheit eines Inertgases wie Stickstoff oder Argon durchgeführt wird.

10. Verfahren nach einem der Ansprüche 1 bis 9, **dadurch gekennzeichnet, dass** ein Katalysator zugegeben wird.

11. Verfahren nach Anspruch 10, **dadurch gekennzeichnet, dass** der Katalysator ausgewählt wird aus den Mineralsäuren oder organischen Säuren, den mineralischen oder organischen Basen, den Katalysatoren auf Zinn- oder Titanbasis.

12. Verfahren nach Anspruch 10, **dadurch gekennzeichnet, dass** der Katalysator Paratoluolsulfonsäure oder Methansulfonsäure ist.

13. Verfahren nach einem der Ansprüche 1 bis 12, **dadurch gekennzeichnet, dass** man eine Säurezahl kleiner oder gleich 3 erhält.

## Claims

1. A one-pot method for the synthesis of estolide esters, **characterised in that** in the absence of solvent a hydroxyacid saturated by a monoalcohol is esterified and that simultaneously an oligomerisation is realized to form estolide esters having a degree of oligomerisation greater than or equal to 3, an acid index less than or equal to 5 and which are saturated and thermo-oxidatively stable.

2. A method according to Claim 1, **characterised in that** the hydroxyacid is selected from the saturated and hydroxylated carboxylic acids.

3. A method according to Claim 2, **characterised in that** the saturated and hydroxylated carboxylic acids are carboxylic acids of plant origin such as 12-hydroxystearic acid.

4. A method according to Claim 1, **characterised in that** the monoalcohol is selected from the branched monoalcohols.

5. A method according to Claim 4, **characterised in that** the alcohol is ethyl-2-hexanol.

6. A method according to Claim 4, **characterised in that** the alcohol is selected from the isoalkyl alcohols.

7. A method according to any one of Claims 1 to 6, **characterised in that** it is realized at a temperature comprised between 100 and 200°C.

8. A method according to Claim 7, **characterised in that** it is realized at a temperature comprised between 140 and 180°C.

9. A method according to any one of Claims 1 to 8, **characterised in that** it is realized in the presence of an inert gas such as nitrogen or argon.

10. A method according to any one of Claims 1 to 9, **characterised in that** a catalyst is added.

11. A method according to Claim 10, **characterised in that** the catalyst is selected from the mineral or organic acids, the mineral or organic bases, the catalysts based on tin or titanium.

12. A method according to Claim 10, **characterised in that** the catalyst is paratoluene sulphonic acid or methane sulphonic acid.

13. A method according to any one of Claims 1 to 12, **characterised in that** an acid index less than or equal to 3 is obtained.
